# EUROPEAN PATENT SPECIFICATION

(11) **EP 3 346 013 B1**
(45) Date of publication and mention of the grant of the patent: **30.12.2020**
(21) Application number: 16841985.1
(22) Date of filing: 01.09.2016
(51) Int. Cl.: C12Q 1/26, C12M 1/34, C12Q 1/37, G01N 27/30, G01N 27/416

(54) **METHOD FOR MEASURING GLYCATED PROTEIN USING COMB ELECTRODE**
VERFAHREN ZUR MESSUNG VON GLYKOSILIERTEM PROTEIN MIT EINER KAMMELEKTRODE
PROCÉDÉ DE MESURE D'UNE PROTÉINE GLYQUÉE À L'AIDE D'UNE ÉLECTRODE À PEIGNE

(30) Priority: 02.09.2015 JP 2015172879
(43) Date of publication of application: 11.07.2018
(73) Proprietor: Ultizyme International Ltd., Meguro Tokyo 152-0013 (JP)
(72) Inventor: UEDA, Yuki, Tokyo 101-8101 (JP); SODE, Koji, Fuchu-shi Tokyo 183-8538 (JP); TSUGAWA, Wakako, Fuchu-shi Tokyo 183-8538 (JP); KURITA, Masaaki, Hiratsuka-shi Kanagawa 254-0076 (JP); NISHIMORI, Takashi, Hiratsuka-shi Kanagawa 254-0076 (JP)
(74) Representative: Mathys & Squire
(86) International application number: PCT/JP2016/075733
(87) International publication number: WO 2017/038956

(56) References cited:
- WO-A1-2014/112569
- JP-A- 2005 512 027
- JP-A- 2007 163 440
- JP-A- 2009 171 874
- JP-A- 2013 500 729
- US-A1- 2002 090 632
- US-A1- 2012 208 226
- STEFANO FERRI ET AL: "Tuning Fructosyl Peptidyl Oxidase into Dehydrogenase and Its Application for the Construction of an Enzyme Electrode", ECS TRANSACTIONS, 1 January 2011 (2011-01-01), pages 113-116, XP55275198, US ISSN: 1938-5862, DOI: 10.1149/1.3571982
- CHAWLA SHEETAL ET AL: "An amperometric hemoglobin A1c biosensor based on immobilization of fructosyl amino acid oxidase onto zinc oxide nanoparticles-polypyrrole film", ANALYTICAL BIOCHEMISTRY, vol. 430, no. 2, 15 November 2012 (2012-11-15), pages 156-162, XP028945647, ISSN: 0003-2697, DOI: 10.1016/J.AB.2012.08.002
- SEUNGSU KIM ET AL: "Construction of engineered fructosyl peptidyl oxidase for enzyme sensor applications under normal atmospheric conditions", BIOTECHNOLOGY LETTERS, SPRINGER NETHERLANDS, DORDRECHT, vol. 34, no. 3, 4 November 2011 (2011-11-04), pages 491-497, XP035011922, ISSN: 1573-6776, DOI: 10.1007/S10529-011-0787-1
- MIKA HATADA ET AL: "Development of enzyme sensor for glycated albumin measurement using screen-printed electrode", ABSTRACTS OF THE 82ND ANNUAL MEETING OF THE ELECTROCHEMICAL SOCIETY OF JAPAN,, 9 March 2015 (2015-03-09), page 1M32, XP009509570,
- YUE HUANG ET AL: "A redox-enzyme-based electrochemical biosensor with a CMOS integrated bipotentiostat", BIOMEDICAL CIRCUITS AND SYSTEMS CONFERENCE, 2009. BIOCAS 2009. IEEE, IEEE, PISCATAWAY, NJ, USA, 26 November 2009 (2009-11-26), pages 29-32, XP031595018, ISBN: 978-1-4244-4917-0
- GUOZHEN LIU ET AL: "An Electrochemical Impedance Immunosensor Based on Gold Nanoparticle-Modified Electrodes for the Detection of HbA1c in Human Blood", ELECTROANALYSIS, vol. 24, no. 7, 27 June 2012 (2012-06-27), pages 1509-1516, XP55582193, US ISSN: 1040-0397, DOI: 10.1002/elan.201200233
- MARA MIRASOLI ET AL: "Recent advancements in chemical luminescence-based lab-on-chip and microfluidic platforms for bioanalysis", JOURNAL OF PHARMACEUTICAL AND BIOMEDICAL ANALYSIS, vol. 87, 1 January 2014 (2014-01-01), pages 36-52, XP55421516, US ISSN: 0731-7085, DOI: 10.1016/j.jpba.2013.07.008
- MIKA HATADA et al.: "Development of enzyme sensor for glycated albumin measurement using screen-printed electrode", Abstracts of the 82nd Annual Meeting of the Electrochemical Society of Japan, 9 March 2015 (2015-03-09), page 1M32, XP009509570,
- MIKA HATADA et al.: "Kushi-gata Denkyoku o Mochiiru Toka Albumin Keisoku-yo Tsukaisute- gata Kokando Koso Sensor no Kaihatsu", Abstracts of the 83rd Annual Meeting of the Electrochemical Society of Japan, 23 March 2016 (2016-03-23), page PS94, XP009509437,
- HATADA, MIKA et al.: "Development of a screen- printed carbon electrode based disposable enzyme sensor strip for the measurement of glycated albumin", Biosens. Bioelectron., vol. 88 , pages 167-173, XP055368892,

## Description

### Technical Field

The present disclosure relates to a method for measuring a glycated protein in a sample and a biosensor for the measurement.

### Background Art

It is known that the blood level of glycated proteins reflects the average blood glucose level over a certain period in the past and the measured value of the blood level can be an important indicator for diagnosis and management of symptoms of diabetes mellitus. As the glycated proteins, hemoglobin A1c (HbA1c), which is the glycated protein of hemoglobin, and glycated albumin (GA), which is the glycated protein of albumin, are known. The blood level of glycated proteins is a stable indicator reflecting the blood glucose level over a long period (several weeks to several months) and is therefore useful for judging whether or not the glycemic control that is performed for diagnosing or treating diabetes mellitus is successful.

As a method for measuring the blood level of a glycated protein, Patent Literature 1 discloses a method using a fructosyl amino acid oxidase (hereinafter, may be referred to as "FAOD"). In the method disclosed in

Patent Literature 1, a sample is pretreated with a protease to degrade a glycated protein into glycated peptides and glycated amino acids before the reaction between the sample and FAOD, and thereby the reaction rate of FAOD is enhanced, and the detection sensitivity for the glycated protein is enhanced. The titer of FAOD can be measured by using a known method, such as a colorimetric method, a fluorescent method, and an electrochemical method.

Furthermore, various procedures, in which about 0.3 to 10 microliters (µL) of a liquid sample is sucked into a sensor unit, the sample is treated with a variety of reagents in the sensor unit, and the reaction is measured to analyze the sample, have been proposed. In particular, a biosensor that can measure the concentration of a specific substance in a sample by utilizing the substrate specificity of an enzyme used as a reagent is suitable for analysis of a biological sample containing a variety of substances and therefore has been used not only as medical test equipment but also in various fields. Patent Literature 2 discloses a known example of application of such a biosensor to measurement of the blood level of a glycated protein. In the biosensor disclosed in Patent Literature 2, FAOD is immobilized on an electrode together with an artificial electron mediator by a water-soluble photocurable resin, and the glycated protein concentration is measured by an electrochemical method.

Furthermore, Patent Literature 3 discloses a method for measuring a blood component, such as glucose, by applying a biosensor including an interdigitated electrode to the measurement. In the method disclosed in Patent Literature 3, the biosensor having an interdigitated electrode oxidizes the blood component by an oxidoreductase and detects the oxidation current due to the reaction product with the electrode to measure the blood component.

### Citation List

### Patent Literature

Patent Literature 1: International Publication No. WO 98/48043
Patent Literature 2: Japanese Patent Laid-Open No. 2009-171874
Patent Literature 3: International Publication No. WO 2014/112569

Huang et al, "A redox-enzyme-based electrochemical biosensor with a CMOS integrated biopotentiostat", BIOMEDICAL CIRCUITS AND SYSTEMS CONFERENCE, 2009. BIOCAS 2009. IEEE, IEEE, PISCATAWAY, NJ, USA, 26 November 2009, pages 29-32 discloses an electrochemical biosensor featuring redox enzymes and a supporting CMOS biopotentiostat.

Guozhen Liu et al. "An electrochemical Impedance Immunosensor based on gold nanoparticle-modified electrodes for the detection of HbAlc in Human Blood", ELECTROANALYSIS, vol. 24, no. 7, 27 June 2012, pages 1509-1516 discloses a label-free immunosensor for the detection of HbAlc based on gold nanoparticle (AuNP)-aryl diazonium salt modified glassy carbon (GC) electrode where transduction is achieved using electrochemical impedance spectroscopy (EIS).

Mara Mirasoli et al. "Recent advancements in chemical luminescence-based lab-on-chip and microfluidic platforms for bioanalysis", JOURNAL OF PHARMACEUTICAL AND BIOMEDICAL ANALYSIS, vol. 87, 1 January 2014, pages 36-52 discloses miniaturization of analytical procedures through microchips, lab-on-a-chip or micro total analysis systems.

### Summary of Invention

### Technical Problem

The methods for measuring glycated proteins disclosed in Patent Literatures 1 and 2 use known methods, such as electrical measuring methods, in measurement of the titer of FAOD and both show insufficient sensitivity and data stability, leading to difficulty in practical application. The measurement method using a biosensor disclosed in Patent Literature 3 relates to measurement of a blood component, such as glucose, and application of the method to measurement of a glycated protein is unknown.

It is an object of the present disclosure to solve these problems and to provide a highly sensitive method for measuring the concentration of a glycated protein in a sample easily and inexpensively.

### Solution to Problem

The present inventors have diligently studied to solve the above-described problems and has found that the concentration of a glycated protein in a sample can be highly sensitively quantified easily and inexpensively, surprisingly, by allowing a combination of an electron mediator and an interdigitated electrode to react with a sample that has been brought into contact with a protease for generating a degradation product from the glycated protein, and the present disclosure has been accomplished.

According to a first aspect of the invention, there is provided a method for measuring a glycated protein in a sample, comprising (1) a step of allowing a sample in which a degradation product has been generated from a glycated protein by a protease to react with fructosyl amino acid oxidase in the presence of 300 mmol/L or more of hexaammineruthenium (III) chloride to generate a reduced hexaammineruthenium (III) chloride; and (2) a step of detecting the reaction state in the step (1) by an electrochemical technique using an interdigitated electrode, wherein the interdigitated electrode has a total area of 1.8 mm² to 4 mm², an interelectrode distance of less than 50 µm, a working electrode width of 5 µm to 50 µm, and a counter electrode width of 5 µm to 100 µm..

Preferably, the composition comprising the fructosyl amino acid oxidase and the hexaammineruthenium (III) chloride is disposed on the interdigitated electrode.

Preferably, the electrochemical technique is a constant potential amperometry.

Preferably, the glycated protein is glycated albumin.

Preferably, concentration of the fructosyl amino acid oxidase in the step (1) is 12 U/mL or more.

According to a further aspect of the invention, there is provided a biosensor for measuring glycated albumin in a sample, comprising (1) a composition comprising 300 mmol/L or more of hexaammineruthenium(III) chloride and 12 U/mL or more of a fructosyl amino acid oxidase and (2) an interdigitated electrode having a total area of 1.8 mm² to 4 mm², an interelectrode distance of less than 50 µm, a working electrode width of 5 µm to 50 µm, and a counter electrode width of 5 µm to 100 µm, wherein the composition comprising the fructosyl amino acid oxidase and the hexaammineruthenium(III) chloride is disposed on the interdigitated electrode; and a degradation product generated from a glycated albumin being in contact with a protease is allowed to react with the fructosyl amino acid oxidase.

### Advantageous Effects of Disclosure

According to the present disclosure, the concentration of a glycated protein in a sample can be highly sensitively quantified easily and inexpensively by allowing a combination of an electron mediator and an interdigitated electrode to react with a sample in which a degradation product has been generated from the glycated protein by a protease.

### Brief Description of Drawings

[Figure 1] Figure 1 is a graph showing a relationship between the sensitivity in measurement of benzyloxycarbonyl-fructosyl lysine and the concentration of a fructosyl amino acid oxidase according to an Example.
[Figure 2] Figure 2 is a graph showing a relationship between the sensitivity in measurement of benzyloxycarbonyl-fructosyl lysine and the concentration of hexaammineruthenium(III) chloride according to an Example.
[Figure 3] Figure 3 is a graph showing a standard curve (printed carbon electrode) in benzyloxycarbonyl-fructosyl lysine measurement according to a Comparative Example.
[Figure 4] Figure 4 is a graph showing a standard curve (interdigitated electrode) in benzyloxycarbonyl-fructosyl lysine measurement according to an Example.
[Figure 5] Figure 5 is a graph showing standard curves in benzyloxycarbonyl-fructosyl lysine measurement using a carbon electrode and an interdigitated electrode on which the reagents according to Example were disposed.
[Figure 6] Figure 6 is a graph showing changes of reaction (printed carbon electrode) over time in measurement of a protease digestion product of glycated albumin according to a Comparative Example.
[Figure 7] Figure 7 is a graph showing changes of reaction (interdigitated electrode) over time in measurement of a protease digestion product of glycated albumin according to an Example.

### Description of Embodiments of the Disclosure

Embodiments of the present disclosure (hereinafter, referred to as "the embodiment") will now be specifically described. However, it should not be understood that the following embodiments are intended to limit the present invention.

The embodiment relates to a method for measuring a glycated protein in a sample, the measuring method comprising:
(1) a step of allowing a sample in which a degradation product has been generated from a glycated protein by a protease to react with an oxidoreductase in the presence of an electron mediator to generate a reduced electron mediator; and
(2) a step of detecting the reaction state in the step (1) by an electrochemical technique using an interdigitated electrode.

The glycated protein to be measured by the method according to the embodiment may be any glycated product of a protein without particular limitation. For example, a substance obtained by forming a Schiff's base by a Maillard reaction between an amino group in a protein and a saccharide and subjecting the Schiff's base to Amadori rearrangement is preferred, but the glycated protein is not limited thereto. Specifically, an example of the glycated protein is glycated albumin.

The "sample in which a degradation product has been generated from a glycated protein by a protease" refers to a sample that has been subjected to a process of bringing a protease into contact with a glycated protein possibly contained in the sample for generating a degradation product of the glycated protein, but whether or not the degradation product has actually been generated is not a matter. Although the sample is one possibly containing a glycated protein, the result of measurement may reveal that the sample does not contain the glycated protein.

The protease may be any protease that can degrade a glycated protein into a substrate of an oxidoreductase without particular limitation, and examples thereof include animal-derived proteases, such as trypsin and chymotrypsin; plant-derived proteases, such as papain and bromelain; and microorganism-derived proteases.

Examples of the microorganism-derived protease include Bacillus-derived proteases, represented by orientase 22BF (manufactured by HBI Enzymes Inc.), etc.; Aspergillus-derived proteases, represented by protease type-XIII (manufactured by Sigma-Aldrich), etc.; Penicillium-derived proteases, represented by PD enzyme (manufactured by Kikkoman Corporation), etc.; Streptomyces-derived proteases, represented by Pronase; Lysobacter-derived proteases, such as Endoproteinase Lys-c (manufactured by Sigma-Aldrich); Yeast-derived proteases, represented by Proteinase A (manufactured by Sigma-Aldrich), etc.; Tritirachium-derived proteases, represented by Proteinase K (manufactured by Sigma-Aldrich), etc.; Thermus-derived proteases, represented by Aminopeptidase T (manufactured by Boehringer Mannheim), etc.; Pseudomonus-derived proteases, represented by Endoproteinase Asp-N (manufactured by Wako Pure Chemical Industries, Ltd.), etc.; and Achromobacter-derived proteases, represented by Lysyl endopeptidase (manufactured by Wako Pure Chemical Industries, Ltd.), etc.. These specific proteases are mere examples, and the microorganism-derived protease is not limited thereto. A preferred example is orientase 22BF (manufactured by HBI Enzymes Inc.).

The electron mediator according to the embodiment is not particularly limited. For example, potassium ferricyanide, m-PMS (1-methoxy-5-methylphenazinium methyl sulfate), and hexaammineruthenium(III) chloride can be used. Preferably, the electron mediator is hexaammineruthenium(III) chloride.

The concentration of the electron mediator in the step (1) is not particularly limited as long as a glycated protein can be measured. In the case of hexaammineruthenium(III) chloride, the concentration is, for example, 300 mmol/L or more. If the concentration is lower than 300 mmol/L, a current necessary for measurement may not be obtained in some cases.

The oxidoreductase according to the embodiment is not particularly limited and is, for example, a fructosyl amino acid oxidase (FAOD) of which the substrate is a protease degradation product of a glycated protein for the concentration measurement. Specifically, fructosyl amino acid oxidases derived from the genus Gibberella, the genus Aspergillus, the genus Candida, the genus Penicillium, the genus Fusarium, the genus Acremonium, or the genus Debaryomyces can be used, but the FAOD is not limited thereto. The concentration of the FAOD in the step (1) is not particularly limited as long as a glycated protein can be measured, and is, for example, 12 U/mL or more, preferably 60 U/mL or more. If the concentration is less than 12 U/mL, a current necessary for measurement may not be obtained in some cases.

The interdigitated electrode in the method according to the embodiment includes working and counter electrodes that are alternately arranged. The shape of the interdigitated electrode is not particularly limited as long as a glycated protein can be measured and the interdigitated electrode has, for example, a total area of 1.8 mm² to 4 mm², an interelectrode distance of less than 50 µm, a working electrode width of 5 µm to 50 µm, and a counter electrode width of 5 µm to 100 µm.

Examples of the method for producing the interdigitated electrode according to the embodiment include the following four methods.
(1) A noble metal film is formed on an electrically insulating base material. A resist is printed in a comb shape on the film by screen printing, and the noble metal film in the portion not covered with the resist is etched. The resist is then removed to form an interdigitated electrode.
(2) A noble metal film is formed on an electrically insulating base material. A resist is applied or pasted on the film and is exposed to light through a photomask to cure the resist in the portion forming an interdigitated electrode. The resist and the noble metal film other than the portion forming an interdigitated electrode are then etched, and the resist in the portion forming an interdigitated electrode is removed to form an interdigitated electrode.
(3) A template cut into an interdigitated electrode pattern to be produced is overlaid on an electrically insulating base material. A noble metal film is formed on the electrically insulating base material through the template, and the template is then removed to form an interdigitated electrode.
(4) A resist is printed by screen printing on an electrically insulating base material in the portion not forming an interdigitated electrode, and a noble metal film is formed on the electrically insulating base material and the resist. The resist and the noble metal film formed on the resist are then removed to form an interdigitated electrode.

Examples of the material for the electrically insulating base material include polyester resin, polyethylene terephthalate, and engineering plastic. Examples of the noble metal include gold, platinum, silver, palladium, ruthenium, iridium, rhodium, and mixtures thereof. However, the method for producing an interdigitated electrode is not limited to those described above.

The electrochemical technique to be used in the method according to the embodiment may be a known method, such as a constant potential method, but is not limited thereto.

An example of the principle of measuring a glycated protein by a constant potential method is as follows. First, a protease is brought into contact with a glycated protein in a sample for generating a degradation product of the glycated protein. Subsequently, the generated degradation product of the glycated protein is oxidized by an oxidoreductase. The oxidation reaction of the degradation product reduces the electron mediator as an electron receptor of the oxidation reaction. Application of a constant potential to the interdigitated electrode reoxidizes the reduced electron mediator and gives a response current. Since the response current intensity correlates with the glycated protein concentration, the concentration of a glycated protein can be measured by measuring the response current value.

In the method of the embodiment, an oxidoreductase is disposed together with an electron mediator on an interdigitated electrode in some cases, but the method is not limited thereto. An electron mediator and an oxidoreductase may be disposed on an interdigitated electrode by a known method. For example, drying for disposition, in which a solution of a mixture of an electron mediator and an oxidoreductase is dropwise applied onto an interdigitated electrode and is then dried, is used, but the method is not limited thereto.

In addition, the embodiment relates to a biosensor for measuring a glycated protein in a sample, the biosensor comprising:
(1) a composition comprising an electron mediator and an oxidoreductase; and
(2) an interdigitated electrode, wherein
   a degradation product generated from a glycated protein by a protease is allowed to react with the oxidoreductase.

The glycated protein to be measured by the biosensor according to the embodiment may be any substance obtained by forming a Schiff's base by a Maillard reaction of an amino group in a protein and a saccharide and subjecting the Schiff's base to Amadori rearrangement, without particular limitation, and the glycated protein is, for example, glycated albumin.

The "sample in which a degradation product has been generated from a glycated protein by a protease" refers to a sample that has been subjected to a process of bringing a protease into contact with a glycated protein possibly contained in the sample for generating a degradation product of the glycated protein, but whether or not the degradation product has actually been generated is not a matter.

The sample is one possibly containing a glycated protein, and the result of measurement may reveal that the sample does not contain the glycated protein. The protease may be any protease that can degrade a glycated protein into a substrate of an oxidoreductase, without particular limitation, and examples thereof include animal-derived proteases, such as trypsin and chymotrypsin; plant-derived proteases, such as papain and bromelain; and microorganism-derived proteases.

Examples of the microorganism-derived protease include Bacillus-derived proteases, represented by orientase 22BF (manufactured by HBI Enzymes Inc.), etc.; Aspergillus-derived proteases, represented by protease type-XIII (manufactured by Sigma-Aldrich), etc.; Penicillium-derived proteases, represented by PD enzyme (manufactured by Kikkoman Corporation), etc.; Streptomyces-derived proteases, represented by Pronase, etc.; Lysobacter-derived proteases, represented by Endoproteinase Lys-c (manufactured by Sigma-Aldrich), etc.; Yeast-derived proteases, represented by Proteinase A (manufactured by Sigma-Aldrich), etc.; Tritirachium-derived proteases, represented by Proteinase K (manufactured by Sigma-Aldrich), etc.; Thermus-derived proteases, represented by Aminopeptidase T (manufactured by Boehringer Mannheim), etc.; Pseudomonus-derived proteases, represented by Endoproteinase Asp-N (manufactured by Wako Pure Chemical Industries, Ltd.), etc.; and Achromobacter-derived proteases, represented by Lysyl endopeptidase (manufactured by Wako Pure Chemical Industries, Ltd.), etc.. These specific proteases are mere examples, and the microorganism-derived protease is not limited thereto. A preferred example is orientase 22BF (manufactured by HBI Enzymes Inc.).

The electron mediator in the biosensor according to the embodiment is not particularly limited. For example, potassium ferricyanide, m-PMS (1-methoxy-5-methylphenazinium methyl sulfate), and hexaammineruthenium(III) chloride can be used. Preferably, the electron mediator is hexaammineruthenium(III) chloride.

The concentration of the electron mediator is not particularly limited as long as a glycated protein can be measured. In the case of hexaammineruthenium(III) chloride, the concentration is set such that the concentration when a sample solution is added to the biosensor is, for example, 300 mmol/L or more. If the concentration is lower than 300 mmol/L, a current necessary for measurement may not be obtained in some cases.

The oxidoreductase in the biosensor according to the embodiment is not particularly limited and is, for example, a fructosyl amino acid oxidase (FAOD) of which the substrate is a protease degradation product of a glycated protein for the concentration measurement. Specifically, fructosyl amino acid oxidases derived from the genus Gibberella, the genus Aspergillus, the genus Candida, the genus Penicillium, the genus Fusarium, the genus Acremonium, and the genus Debaryomyces can be used, but the FAOD is not limited thereto. The concentration of the FAOD is not particularly limited as long as a glycated protein can be measured, and is set such that the concentration when a sample solution is added to the biosensor is, for example, 12 U/mL or more. If the concentration is less than 12 U/mL, a current necessary for measurement may not be obtained in some cases.

The interdigitated electrode in the biosensor according to the embodiment includes alternately arranged working and counter electrodes. The shape of the interdigitated electrode is not particularly limited as long as a glycated protein can be measured, and the interdigitated electrode has, for example, a total area of 1.8 mm² to 4 mm², an interelectrode distance of less than 50 µm, a working electrode width of 5 µm to 50 µm, and a counter electrode width of 5 µm to 100 µm.

Examples of the method for producing the interdigitated electrode of the biosensor according to the embodiment include the following four methods:
(1) A noble metal film is formed on an electrically insulating base material. A resist is printed in a comb shape on the film by screen printing, and the noble metal film in the portion not covered with the resist is etched. The resist is then removed to form an interdigitated electrode.
(2) A noble metal film is formed on an electrically insulating base material. A resist is applied or pasted on the film and is exposed to light through a photomask to cure the resist in the portion forming an interdigitated electrode. The resist and the noble metal film other than the portion forming an interdigitated electrode are then etched, and the resist in the portion forming an interdigitated electrode is removed to form an interdigitated electrode.
(3) A template cut into an interdigitated electrode pattern to be produced is overlaid on an electrically insulating base material. A noble metal film is formed on the electrically insulating base material through the template, and the template is then removed to form an interdigitated electrode.
(4) A resist is printed by screen printing on an electrically insulating base material in the portion not forming an interdigitated electrode, and a noble metal film is formed on the electrically insulating base material and the resist. The resist and the noble metal film formed on the resist are then removed to form an interdigitated electrode.

Examples of the material for the electrically insulating base material include polyester resin, polyethylene terephthalate, and engineering plastic. Examples of the noble metal include gold, platinum, silver, palladium, ruthenium, iridium, rhodium, and mixtures thereof. However, the method for producing an interdigitated electrode is not limited to those described above.

The biosensor according to the embodiment detects the reaction state between a degradation product of a glycated protein and an oxidoreductase by an electrochemical technique. The electrochemical technique to be used may be a known method, such as a constant potential method, but is not limited thereto.

An example of the principle of measuring a glycated protein by a constant potential method is as follows. First, a protease is brought into contact with a glycated protein in a sample for generating a degradation product of the glycated protein. Subsequently, the generated degradation product of the glycated protein is oxidized by the oxidoreductase of a biosensor. The oxidation reaction of the degradation product reduces the electron mediator as an electron receptor of the oxidation reaction. Application of a constant potential to the interdigitated electrode reoxidizes the reduced electron mediator and gives a response current. Since the response current intensity correlates with the glycated protein concentration, the concentration of a glycated protein can be measured by measuring the response current value.

In the biosensor according to the embodiment, an oxidoreductase is disposed together with an electron mediator on an interdigitated electrode in some cases, but the biosensor is not limited thereto. An electron mediator and an oxidoreductase may be disposed on an interdigitated electrode by a known method. For example, drying for disposition, in which a solution of a mixture of an electron mediator and an oxidoreductase is dropwise applied onto an interdigitated electrode and is then dried, is used, but the method is not limited thereto.

The embodiment further relates to a biosensor for measuring glycated albumin in a sample, the biosensor comprising:
(1) a composition comprising hexaammineruthenium(III) chloride in amounts set to give a concentration of 300 mmol/L or more when a sample solution is added thereto and a fructosyl amino acid oxidase in amounts set to give a concentration of 12 U/mL or more when a sample solution is added thereto; and
(2) an interdigitated electrode having a total area of 1.8 mm² to 4 mm², an interelectrode distance of less than 50 µm, a working electrode width of 5 µm to 50 µm, and a counter electrode width of 5 µm to 100 µm, wherein the composition comprising the fructosyl amino acid oxidase and the hexaammineruthenium(III) chloride is disposed on the interdigitated electrode; and a degradation product generated from the glycated protein by a protease is allowed to react with the fructosyl amino acid oxidase.

### Examples

An Example measuring the concentration of glycated albumin (GA) in a sample by using a fructosyl amino acid oxidase (FAOD) will now be described, but it should be noted that the sample to be subjected to measurement or the enzyme is not limited thereto. The measurement of glycated albumin is based on the following principle. GA contained in a sample is digested with a protease to release fructosyl lysine (ε-FK) having a structure in which the amino group on the ε-position of a lysine residue is glycated. The free ε-FK is oxidized by FAOD. The oxidation reaction of ε-FK reduces an artificial electron receptor (mediator) serving as an electron receptor of the oxidation reaction. Application of a constant potential to the interdigitated electrode reoxidizes the reduced mediator and gives a response current. Since the response current intensity correlates with the GA concentration, the concentration of GA can be measured by measuring the response current value. In the following Examples, as a synthetic substrate of ε-FK, benzyloxycarbonyl-fructosyl lysine (Z-FK) in which the amino group on the α-position was masked by a benzyloxycarbonyl group, was used.

### (Example 1) Optimization of FAOD concentration

### <Reagent (final concentration)>

10 mmol/L potassium phosphate buffer (PPB) (pH 8.0)
FAOD (1.2 to 240 U/mL)
Hexaammineruthenium(III) chloride (100 mmol/L)

### <Substrate (final concentration)>

Z-FK solution (0 to 500 µmol/L)

### <Used electrode>

Interdigitated electrode (the interdigitated electrode, described in International Publication No. WO 2014/112569, developed by Tanaka Kikinzoku Kogyo K.K.)

FAOD was dissolved in 40 mmol/L PPB to give concentrations of 4.8, 9.6, 24, 48, 96, 192, 240, 480, 640, and 960 U/mL. Hexaammineruthenium(III) chloride was dissolved in purified water to give a concentration of 400 mmol/L. Z-FK was dissolved in purified water to give concentrations of 100, 200, 600, and 1000 µmol/L.

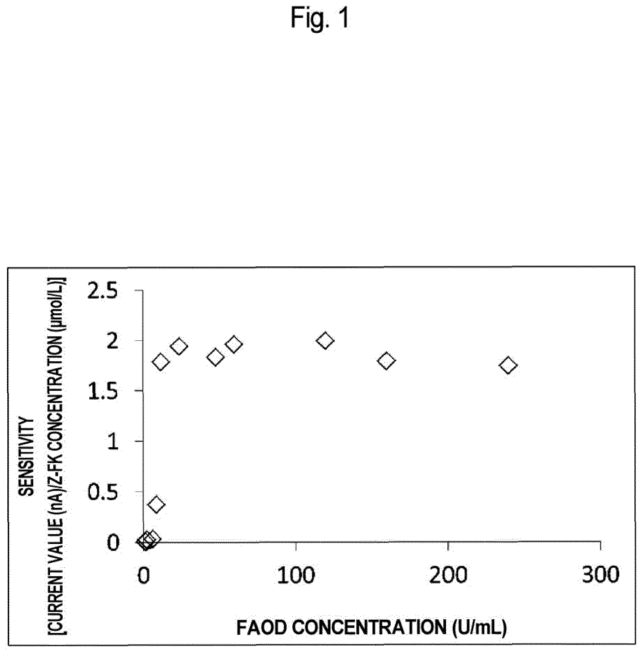

The FAOD solutions (each 3 µL) were respectively mixed with the hexaammineruthenium(III) chloride aqueous solution (3 µL), and the mixtures were then respectively mixed with 6 µL of the Z-FK aqueous solution or purified water. On this occasion, the final concentration of FAOD was 1.2, 2.4, 6, 9, 12, 24, 48, 60, 120, 160, or 240 U/mL; the final concentration of hexaammineruthenium(III) chloride was 100 mmol/L; and the final concentration of Z-FK was 0, 50, 100, 300, or 500 µmol/L. The mixed solutions (each 3 µL) were respectively dropwise added onto the working and counter electrodes of an interdigitated electrode. After a reaction at room temperature for 60 seconds, constant potential measurement was performed under application of a voltage of 100 mV, and current values after 5 seconds were compared to one another. The results of analysis of the relationship between the sensitivity (current value/Z-FK concentration) and the FAOD concentration showed an FAOD concentration-dependent increase of the sensitivity, as shown in Fig. 1. The results of observation suggested that a final concentration of FAOD of 12 U/mL or more stabilizes the sensitivity and that a final concentration of 60 U/mL or more provides a sufficient reaction efficiency.

### (Example 2) Optimization of mediator concentration

### <Reagent (final concentration)>

10 mmol/L potassium phosphate buffer (PPB) (pH 8.0)
FAOD (12 U/mL)
Hexaammineruthenium(III) chloride (1 to 400 mmol/L)

### <Substrate (final concentration)>

Z-FK solution (0 to 500 µmol/L)

### <Used electrode>

Interdigitated electrode (the interdigitated electrode, described in International Publication No. WO 2014/112569, developed by Tanaka Kikinzoku Kogyo K.K.)

FAOD was dissolved in 120 mmol/L PPB to give a concentration of 144 U/mL. Hexaammineruthenium(III) chloride was dissolved in purified water to give concentrations of 4, 40, 200, 400, 600, 800, 1200, and 1600 mmol/L. Z-FK was dissolved in purified water to give concentrations of 75, 150, 450, and 750 µmol/L.

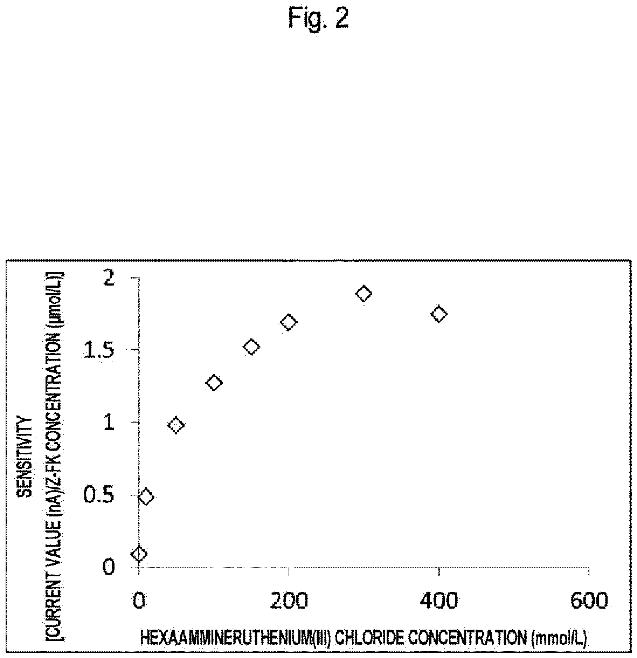

The hexaammineruthenium(III) chloride aqueous solutions (each 3 µL) were respectively mixed with the FAOD solution (1 µL), and the mixtures were then respectively mixed with 8 µL of the Z-FK aqueous solution or purified water. On this occasion, the final concentration of FAOD was 12 U/mL; the final concentration of hexaammineruthenium(III) chloride was 1, 10, 50, 100, 150, 300, or 400 mmol/L; and the final concentration of Z-FK was 0, 50, 100, 300, or 500 µmol/L. The mixed solutions (each 3 µL) were respectively dropwise added onto the working and counter electrodes of an interdigitated electrode. After a reaction at room temperature for 60 seconds, constant potential measurement was performed under application of a voltage of 100 mV, and current values after 5 seconds were compared to one another. The results of analysis of the relationship between the sensitivity (current value/Z-FK concentration) and the hexaammineruthenium(III) chloride concentration showed a hexaammineruthenium(III) chloride concentration-dependent increase of the sensitivity, as shown in Fig. 2. The results of observation revealed that a final concentration of hexaammineruthenium(III) chloride of 300 mmol/L or more is preferred.

### (Example 3) Comparison of printed carbon electrode and interdigitated electrode

### <Reagent (final concentration)>

10 mmol/L potassium phosphate buffer (PPB) (pH 8.0)
FAOD (60 U/mL)
Hexaammineruthenium(III) chloride (300 mmol/L)

### <Substrate (final concentration)>

Z-FK aqueous solution (0 to 500 µmol/L)

### <Used electrode>

Interdigitated electrode (the interdigitated electrode, described in International Publication No. WO 2014/112569, developed by Tanaka Kikinzoku Kogyo K.K.)

Printed carbon electrode (manufactured by BioDevice Technology, Ltd.)

FAOD was dissolved in 120 mmol/L PPB to give a concentration of 720 U/mL. Hexaammineruthenium(III) chloride was dissolved in purified water to give a concentration of 1200 mmol/L. Z-FK was dissolved in purified water to give concentrations of 75, 150, 450, and 750 µmol/L.

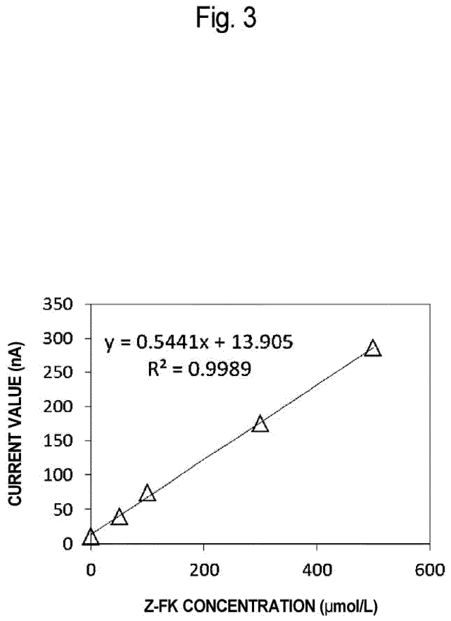

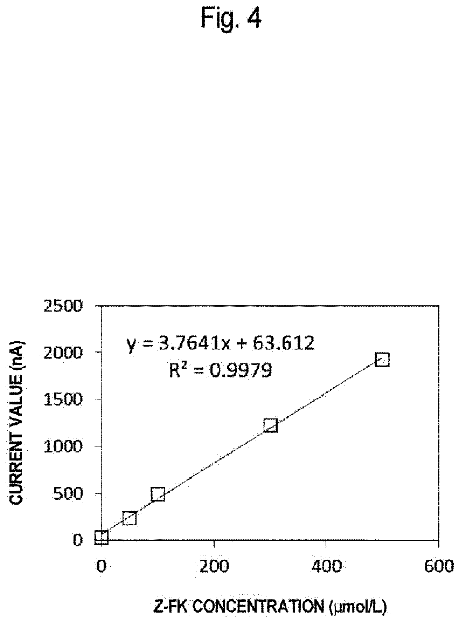

The FAOD solution (1 µL) was mixed with the hexaammineruthenium(III) chloride aqueous solution (3 µL). The Z-FK aqueous solutions (each 8 µL) were respectively mixed with the mixture. The mixed solutions (each 3 µL) were respectively dropwise added onto an interdigitated electrode and a printed carbon electrode. After a reaction for 60 seconds, constant potential measurement was performed under application of a voltage of 100 mV, and current values after 5 seconds (interdigitated electrode) and after 20 seconds (printed carbon electrode) were compared to one another. The results of analysis of the correlation between the Z-FK concentration and the current value demonstrated that the slope of the calibration curve in the Z-FK measurement with the interdigitated electrode shown in Fig. 4 was about 7 times compared to the printed carbon electrode shown in Fig. 3. In addition, the current density observed in the use of the interdigitated electrode was higher than that observed in the use of the printed carbon electrode. That is, when the interdigitated electrode and the printed carbon electrode were compared for the value obtained by dividing the observed current value by the area of the working electrode, i.e., the current density, for example, in a Z-FK concentration of 500 µmol/L, which is the highest concentration in this measurement, the current densities were 1.91 µA/mm² and 119 nA/mm², respectively, and the current density observed in the interdigitated electrode was about 16 times that observed in the printed carbon electrode. Thus, it was revealed that the application of an interdigitated electrode can achieve high sensitivity.

### (Example 4) Arrangement of reagent on interdigitated electrode

### <Reagent (final concentration)>

10 mmol/L potassium phosphate buffer (PPB) (pH 8.0)
FAOD (60 U/mL)
Hexaammineruthenium(III) chloride (300 mmol/L)
Sucrose (0.25%) manufactured by Wako Pure Chemical Industries, Ltd.

### <Substrate (final concentration)>

Z-FK aqueous solution (0 to 500 µmol/L)

### <Used electrode>

Interdigitated electrode (the interdigitated electrode, described in International Publication No. WO 2014/112569, developed by Tanaka Kikinzoku Kogyo K.K.)

Printed carbon electrode (manufactured by BioDevice Technology, Ltd.)

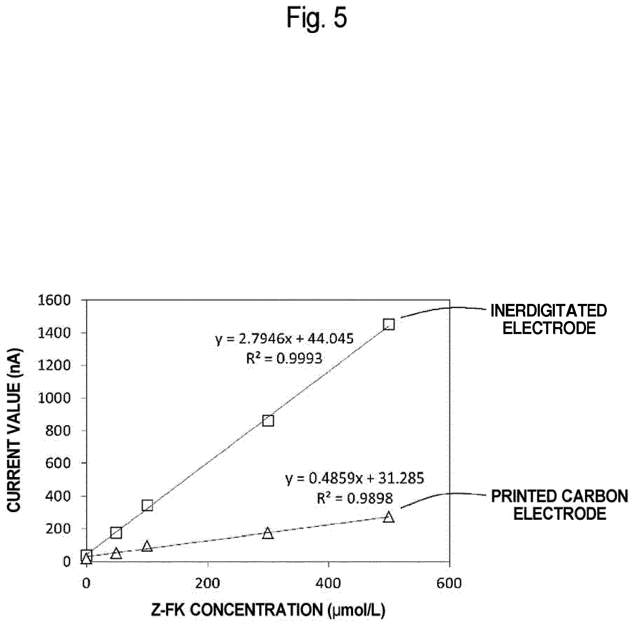

FAOD was dissolved in 120 mmol/L PPB to give a concentration of 720 U/mL. Hexaammineruthenium(III) chloride was dissolved in purified water to give a concentration of 1200 mmol/L. Sucrose was dissolved in purified water to give a concentration of 0.375%. Z-FK was dissolved in purified water to give final concentrations of 50, 100, 300, and 500 µmol/L. The FAOD solution (1 µL), the hexaammineruthenium(III) chloride aqueous solution (3 µL), and the sucrose (8 µL) were mixed. An interdigitated electrode and a printed carbon electrode each equipped with a cover were allowed to absorb the mixture in an amount of 0.8 µL and an amount of 1.3 µL, respectively, and were dried in a thermostatic incubator at 25°C for 30 minutes to produce sensors having FAOD disposed. The produced sensors having FAOD disposed were allowed to absorb the Z-FK solution in an amount of 0.8 µL and in an amount of 1.3 µL, respectively. After a reaction for 60 seconds, constant potential measurement was performed under application of a voltage of 100 mV, and current values after 5 seconds (interdigitated electrode) and after 20 seconds (printed carbon electrode) were compared to one another. The results of analysis of the correlation between the Z-FK concentration and the current value demonstrated, as shown in Fig. 5, that the slope of the calibration curve in the Z-FK measurement using the interdigitated electrode was about 6 times compared to that obtained using the printed carbon electrode. In addition, the current density observed in the use of the interdigitated electrode was higher than that in the use of the printed carbon electrode. That is, when the interdigitated electrode and the printed carbon electrode were compared for the value obtained by dividing the observed current value by the area of the working electrode, i.e., the current density, for example, in a Z-FK concentration of 500 µmol/L, which is the highest concentration in this measurement, the current densities were 1.44 µA/mm² and 114 nA/mm², respectively, and the current density observed in the interdigitated electrode was about 13 times that observed in the printed carbon electrode. A sensor capable of performing highly sensitive measurement was able to be produced by applying an interdigitated electrode.

### (Example 5) Measurement of protease digestion product of glycated albumin

### <Sample>

Glycated albumin (low and high concentrations)

### <Reagent 1 (final concentration)>

Orientase 22BF (manufactured by HBI Enzymes Inc.) (50 mg/mL)

### <Reagent 2 (final concentration)>

10 mmol/L potassium phosphate buffer (PPB) (pH 8.0)
FAOD (60 U/mL)
Hexaammineruthenium(III) chloride (300 mmol/L)
Sucrose (0.25%) manufactured by Wako Pure Chemical Industries, Ltd.

### <Used electrode>

Interdigitated electrode (the interdigitated electrode, described in International Publication No. WO 2014/112569, developed by Tanaka Kikinzoku Kogyo K.K.)

Printed carbon electrode (manufactured by BioDevice Technology, Ltd.)

Orientase 22BF (manufactured by HBI Enzymes Inc.) was dissolved in purified water to give a concentration of 50 mg/mL. Glycated albumin (low or high concentration) and the orientase 22BF solution were mixed at a volume ratio of 1:1, and were allowed to react with each other at 37°C for 1 hour to produce a protease digestion product of glycated albumin. Furthermore, FAOD was dissolved in 120 mmol/L PPB to give a concentration of 720 U/mL. Hexaammineruthenium(III) chloride was dissolved in purified water to give a concentration of 1200 mmol/L. Sucrose was dissolved in purified water to give a concentration of 0.375%. Z-FK was dissolved in purified water to give final concentrations of 50, 100, 300, and 500 µmol/L.

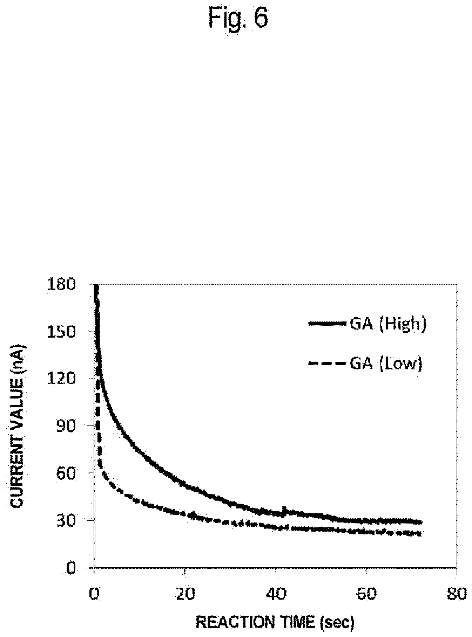

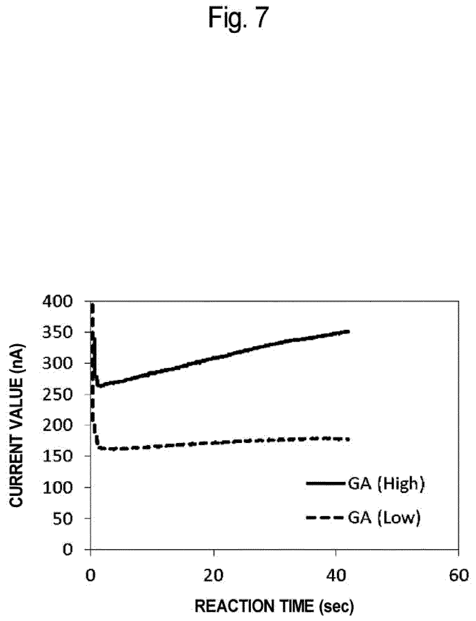

The FAOD solution (1 µL), the hexaammineruthenium(III) chloride aqueous solution (3 µL), and the sucrose (8 µL) were mixed. An interdigitated electrode and a printed carbon electrode each equipped with a cover were allowed to absorb the mixture in an amount of 0.8 µL and an amount of 1.3 µL, respectively, and were dried in a thermostatic incubator at 25°C for 30 minutes to produce sensors each having FAOD disposed. The sensors having FAOD disposed were allowed to absorb the protease digestion product in an amount of 0.8 µL (interdigitated electrode) and in an amount of 1.3 µL (printed carbon electrode), respectively. After a reaction for 60 seconds, constant potential measurement was performed under application of a voltage of 100 mV, and current values after 5 seconds (interdigitated electrode) and after 20 seconds (printed carbon electrode) were compared to one another. The results of analysis of the difference of the current values in measurement of glycated albumin (low and high concentrations) were that the current value difference in the interdigitated electrode was 104 nA after 5 seconds as shown in Fig. 7, whereas the current value difference in the printed carbon electrode was 21 nA after 20 seconds as shown in Fig. 6. It was demonstrated that high-sensitive measurement is possible by application of an interdigitated electrode also in glycated albumin measurement, compared to a conventional printed carbon electrode.

### Industrial Applicability

As described above, according to the method for measuring the concentration of a glycated protein or the biosensor of the present disclosure, high-sensitive quantification of the concentration of a glycated protein in a sample is possible by easy operation and within a short time. It is accordingly possible to provide a simple and inexpensive analytical instrument that can be used, for example, in a blood test in the clinical diagnostic field, by applying the present disclosure. Since the blood level of a glycated protein can be an important indicator for diagnosis and management of symptoms of diabetes mellitus, such an analytical instrument can be expected as a useful means for diagnosis and management of symptoms of diabetes mellitus.

## Claims

1. A method for measuring a glycated protein in a sample, comprising:
(1) a step of allowing a sample in which a degradation product has been generated from a glycated protein by a protease to react with fructosyl amino acid oxidase in the presence of 300 mmol/L or more of hexaammineruthenium (III) chloride to generate a reduced hexaammineruthenium (III) chloride; and
(2) a step of detecting the reaction state in the step (1) by an electrochemical technique using an interdigitated electrode, wherein the interdigitated electrode has a total area of 1.8 mm² to 4 mm², an interelectrode distance of less than 50 µm, a working electrode width of 5 µm to 50 µm, and a counter electrode width of 5 µm to 100 µm..

2. The measuring method according to Claim 1, wherein a composition comprising the fructosyl amino acid oxidase and the hexaammineruthenium (III) chloride is disposed on the interdigitated electrode.

3. The measuring method according to Claim 1 or Claim 2, wherein the electrochemical technique is a constant potential amperometry.

4. The measuring method according to any one of Claims 1 to 3, wherein the glycated protein is glycated albumin.

5. The measuring method according to any one of Claims 1 to 4, wherein a concentration of the fructosyl amino acid oxidase in the step (1) is 12 U/mL or more.

6. A biosensor for measuring glycated albumin in a sample, comprising:
(1) a composition comprising 300 mmol/L or more of hexaammineruthenium(III) chloride and 12 U/mL or more of a fructosyl amino acid oxidase; and
(2) an interdigitated electrode having a total area of 1.8 mm² to 4 mm², an interelectrode distance of less than 50 µm, a working electrode width of 5 µm to 50 µm, and a counter electrode width of 5 µm to 100 µm, wherein
the composition comprising the fructosyl amino acid oxidase and the hexaammineruthenium(III) chloride is disposed on the interdigitated electrode; and a degradation product generated from a glycated albumin being in contact with a protease is allowed to react with the fructosyl amino acid oxidase.

## Patentansprüche

1. Verfahren zur Messung eines glykierten Proteins in einer Probe, umfassend:
(1) einen Schritt zum Ermöglichen einer Probe, in der ein Abbauprodukt aus einem glykierten Protein mittels einer Protease erzeugt wurde, mit Fruktosylaminosäureoxidase in Gegenwart von 300 mmol/l oder mehr von Hexaamminruthenium(III)-chlorid zum Erzeugen eines reduzierten Hexaamminruthenium(III)-chlorids zu reagieren; und
(2) einen Schritt zum Nachweis des Reaktionszustands im Schritt (1) mittels eines elektrochemischen Verfahrens mit einer interdigitierten Elektrode, wobei die interdigitierte Elektrode eine Gesamtfläche von 1,8 mm² bis 4 mm², einen Interelektrodenabstand von kleiner als 50 µm, eine Breite der Arbeitselektrode von 5 µm bis 50 µm und eine Breite der Gegenelektrode von 5 µm bis 100 µm aufweist.

2. Messverfahren nach Anspruch 1, wobei eine Zusammensetzung, umfassend die Fruktosylaminosäureoxidase und das Hexaamminruthenium(III)-chlorid, auf der interdigitierten Elektrode abgelagert ist.

3. Messverfahren nach Anspruch 1 oder Anspruch 2, wobei das elektrochemische Verfahren eine Amperometrie bei konstantem Potential ist.

4. Messverfahren nach einem der Ansprüche 1 bis 3, wobei das glykierte Protein glykiertes Albumin ist.

5. Messverfahren nach einem der Ansprüche 1 bis 4, wobei eine Konzentration der Fruktosylaminosäureoxidase im Schritt (1) 12 E/ml oder mehr beträgt.

6. Biosensor zur Messung von glykiertem Albumin in einer Probe, umfassend:
(1) eine Zusammensetzung, umfassend 300 mmol/l oder mehr von Hexaamminruthenium(III)-chlorid und 12 E/ml oder mehr von einer Fruktosylaminosäureoxidase; und
(2) eine interdigitierte Elektrode mit einer Gesamtfläche von 1,8 mm² bis 4 mm², einem Interelektrodenabstand von kleiner als 50 µm, einer Breite der Arbeitselektrode von 5 µm bis 50 µm und einer Breite der Gegenelektrode von 5 µm bis 100 µm, wobei
die Zusammensetzung, umfassend die Fruktosylaminosäureoxidase und das Hexaamminruthenium(III)-chlorid, auf der interdigitierten Elektrode abgelagert ist; und einem Abbauprodukt, erzeugt aus einem glykierten Albumin, das sich in Kontakt mit einer Protease befindet, ermöglicht wird, mit der Fruktosylaminosäureoxidase zu reagieren.

## Revendications

1. Procédé de mesure d'une protéine glyquée dans un échantillon, comprenant :
(1) une étape consistant à laisser un échantillon dans lequel un produit de dégradation a été produit à partir d'une protéine glyquée par une protéase réagir avec une fructosyl-amine oxydase en présence de 300 mmol/l ou plus de chlorure d'hexaammineruthénium (III) pour produire un chlorure d'hexaammineruthénium (III) réduit ; et
(2) une étape de détection de l'état de réaction à l'étape (1) par une technique électrochimique utilisant une électrode interdigitée, l'électrode interdigitée ayant une surface totale de 1,8 mm² à 4 mm², un espace interélectrode inférieur à 50 µm, une largeur utile d'électrode de 5 µm à 50 µm et une largeur de contre-électrode de 5 µm à 100 µm.

2. Procédé de mesure selon la revendication 1, dans lequel une composition comprenant la fructosyl-amine oxydase et le chlorure d'hexaammineruthénium (III) est disposée sur l'électrode interdigitée.

3. Procédé de mesure selon la revendication 1 ou la revendication 2, dans lequel la technique électrochimique est une ampérométrie à potentiel constant.

4. Procédé de mesure selon l'une quelconque des revendications 1 à 3, dans lequel la protéine glyquée est une albumine glyquée.

5. Procédé de mesure selon l'une quelconque des revendications 1 à 4, dans lequel une concentration de la fructosyl-amine oxydase à l'étape (1) est de 12 U/ml ou plus.

6. Biocapteur conçu pour mesurer une albumine glyquée dans un échantillon, comprenant :
(1) une composition comprenant 300 mmol/l ou plus de chlorure d'hexaammineruthénium (III) et 12 U/ml ou plus d'une fructosyl-amine oxydase ; et
(2) une électrode interdigitée ayant une surface totale de 1,8 mm² à 4 mm², un espace interélectrode inférieur à 50 µm, une largeur utile d'électrode de 5 µm à 50 µm et une largeur de contre-électrode de 5 µm à 100 µm, dans lequel
la composition comprenant la fructosyl-amine oxydase et le chlorure d'hexaammineruthénium (III) est disposée sur l'électrode interdigitée ; et on laisse réagir avec la fructosyl-amine oxydase un produit de dégradation produit par le contact d'une albumine glyquée avec une protéase.
